# EUROPEAN PATENT APPLICATION

(11) **EP 3 053 461 A1**
(43) Date of publication of application: **10.08.2016**
(21) Application number: 15746168.2
(22) Date of filing: 30.01.2015
(51) Int. Cl.: A41D 11/00

(54) **BABY GARMENT**

(30) Priority: 06.02.2014 ES 201430162
(71) Applicant: Garcia Perez, Alejandro, 29730 Rincón de la Victoria (Málaga) (ES)
(72) Inventor: Garcia Perez, Alejandro, 29730 Rincón de la Victoria (Málaga) (ES)
(74) Representative: Lorente Berges, Ana
(86) International application number: PCT/ES2015/070068
(87) International publication number: WO 2015/118202

(57) **Abstract**

An article of clothing for babies that helps relieve infant colic. According to a first embodiment, it comprises an abdominal area, and it also comprises a compartment (2) located in the abdominal area, which is configured to house an electronic device, or a first thermal bag, to help alleviate infant colic. According to a second embodiment, it comprises an abdominal area, and a compartment (2) located in the abdominal area, and a non-removable thermal bag housed inside the compartment (2) to help relieve infant colic.

## Description

### OBJECT OF THE INVENTION

The present invention is applicable in the technological field of clothing. Also, the invention can be applied in the field of treatment of digestive disorders such as colic or abdominal distress, especially in infants.

Specifically, the object of the present invention relates to a garment for babies, preferably for infants aged zero to four months, whose main purpose is to help relieve colic naturally.

### BACKGROUND OF THE INVENTION

Currently there are several products, which are dedicated to provide a sense of well-being in the baby for a certain time, helping to overcome the typical colic, caused by gases that accumulate in the intestinal tract due to various reasons, for example:
- Nourishment of the infant provided through the mother; such as milk, caffeine, nuts, chocolate, carbonated beverages, food additives, as well as the consumption of cold foods, or foods difficult to digest, or intolerance to proteins in cow's milk.
- Feeding the infant too fast (for less than twenty minutes), over feeding, and feeding too frequently, which produces stagnation of the food in the stomach and intestines.
- The consumption of antibiotics.
- Emotional factors: There are cases of traumatic experiences of the mother prior to giving birth (her spouse's death, a traffic accident, etc.) that have been lived and assimilated by the infant. A difficult or a cesarean birth can also cause emotional trauma in the baby.
- Environmental stimuli, such as exposure of the abdomen to wind or cold weather, audiovisual equipment, and environmental pollution, which is continuously increasing.

The products mentioned herein may be, on the one hand, bags and stuffed animals that include a filling of various types of seed kernels, herbs, aromatic oils, or self-heating gels, generally based on natural medicine. In this case, the product with the filling inside is usually heated slightly in the microwave for a few minutes until the filling acquires a temperature slightly higher than the human body. Subsequently, the product is applied to the baby's belly, so that the warmed filling provides the baby with a gentle heat for about one hour (generally until the baby is better) to help alleviate his or her ailments.

The elements of this type that are known today have, as noted above, the shape of a bag or pouch, and sometimes a stuffed animal for the baby to cuddle and feel the pleasant effect that is proposed.

The products can also be, on the other hand, electrical heating devices, and electric massage devices.

The problem of the products described above is that infants of that age do not yet have the motor functions that would allow them to hold the stuffed animal or pouch by themselves, or the electrical heating or massage devices, and even less while sleeping.

### DESCRIPTION OF THE INVENTION

The present invention solves the aforementioned drawback keeping said bag or pouch, or electrical device in contact with the infant's belly at all times, and more particularly while sleeping. For this, a textile garment is described, comprised of an abdominal portion having a compartment, such as a pocket, suitable to place in it the selected item, either a bag with seeds, herbs, or other natural products, an electronic device, or self-heating gel, preventing this element from falling, and therefore failing to comply with the function for which it is intended: to help avoid (or lessen) infant colic.

The compartment defined in said garment consists of a pocket, accessible from the outside of the compartment, and preferably provided with closure means consisting of a single button, some ribbons, or Velcro strips. This pocket will have the appropriate measurements to easily place in it the small cloth bag or other device for heating measures, and thus help relieve the infant wearer of the garment.

This design favorably solves the problem of keeping the product on the baby's abdomen, where thanks to the heat generated by the small bag, it will help relieve the infant's colic

### DESCRIPTION OF THE DRAWINGS

To complement the description being made, and in order to assist for a better understanding of the characteristics of the invention according to an example, preferably from a practical embodiment thereof, as an integral part of said description, herein is a set of drawings that are illustrative and not limitative in character, which show the following:

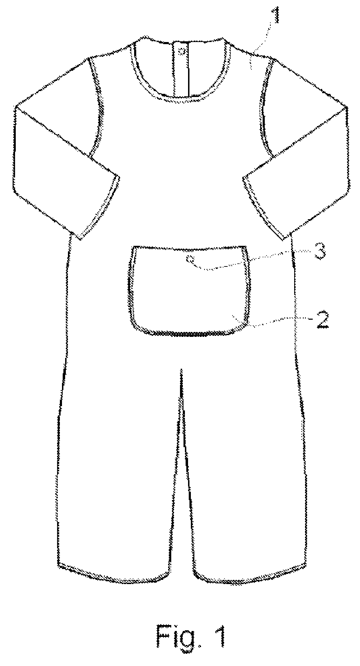

**Figure 1** shows a general view in elevation of a baby's garment made according to the present invention.

### PREFERRED EMBODIMENT OF THE INVENTION

Following is disclosed, with the help of the only Figure 1 aforementioned, a more detailed description of an example of the preferred embodiment of the garment for infants which is the subject of the present invention.

As can be seen in Figure 1, it is a garment (1) for babies that in general has the configuration of pajamas, a t-shirt, or a sweater, or the like, and comprises an abdominal area which is provided with a compartment (2), which in figure 1 is shown, for example, like a pocket, which covers the central portion of the abdominal area, at the height of the intestines, where the compartment (2) incorporates an opening, either at the top or the side, which allows one to place inside a thermal bag or an electronic device, fit to help relieve infant colic.

As an example, the following approximate dimensions are provided for the pocket:
Size 00: 11 cm wide x 9.80 cm high
Size 0: 12 cm wide x 10.40 cm high
Size 3: 13cm wide x 11 cm high

The thermal bag can contain a filler comprised of a combination of seeds, kernels, herbs and natural oils that when heated in a microwave alleviates the babies, or may also include other products such as self-heating gels.

According to a first embodiment illustrated by Figure 1, the compartment (2) has a means of closure (3) which may include buttons, zipper, Velcro strips, adhesives, flap closures, etc. In Figure 1 a means of closure (3) represented includes a button.

According to an alternative embodiment, not shown, the compartment (2) accommodates the thermal bag in a non-removable manner; for example, in an inaccessible manner in the interior of the compartment (2), where it is not possible to access the thermal bag without damaging the garment. One example is that the compartment (2) is closed irreversibly, for example, by sewing or heat sealing, with the thermal bag inside the compartment (2).

## Claims

1. Baby's Clothing or a garment for infants (1) is comprised of an abdominal area, and the garment (1) is **characterized by** also including a compartment (2) located in the abdominal area, and configured to house an electronic device, or a first thermal bag, to help relieve infant colic.

2. Baby's Clothing or a garment for infants (1), according to claim 1, **characterized in that** the compartment (2) comprises an opening to introduce the first thermal bag or the electronic device.

3. Baby's Clothing or a garment for infants (1), according to claim 2, **characterized in that** the opening is located at the top or on a side of the compartment (2).

4. Baby's Clothing or a garment for infants (1), according to any one of claims 1 -3, **characterized in that** the compartment (2) further comprises a closure means for inserting and retaining the first thermal bag or the electronic device in said compartment (2).

5. Baby's Clothing or a garment for infants (1), according to any one of claims 1 -4, **characterized in that** the compartment (2) is located in a central portion of the abdominal area.

6. Baby's Clothing or a garment for infants (1), according to any one of claims 1 -5, **characterized in that** the compartment (2) is configured as a pocket (2).

7. Baby's Clothing or a garment for infants (1), comprising an abdominal area; the garment (1) is also **characterized by** further including:
- A compartment (2) located in the abdominal area; and
- A non-removable thermal bag housed inside the compartment (2) to relieve infant colic.

8. Baby's Clothing or a garment for infants (1), according to claim 7, **characterized in that** the heat pack is integrated inside the compartment (2) so that it is not accessible without damaging the garment (1).

9. Baby's Clothing or a garment for infants (1), according to claim 7, **characterized in that** the thermal bag houses a filler comprising a combination of seeds, kernels, herbs and natural oils, and / or self-heating gels.
